# EUROPEAN PATENT APPLICATION

(11) **EP 4 534 980 A1**
(43) Date of publication of application: **09.04.2025**
(21) Application number: 22945044.0
(22) Date of filing: 14.12.2022
(51) Int. Cl.: G01N 21/65, G01N 33/48, G01J 3/44, B82Y 15/00, B82Y 20/00, B82Y 40/00

(54) **SPECTROSCOPIC ANALYSIS SUBSTRATE INCLUDING POLYCRYSTALLINE STRUCTURES AND MANUFACTURING METHOD THEREOF**

(30) Priority: 31.05.2022 KR 20220066874
(71) Applicant: KOREA INSTITUTE OF MATERIALS SCIENCE, Changwon-si, Gyeongsangnam-do 51508 (KR)
(72) Inventor: JUNG, Ho-sang, Changwon-si Gyeongsangnam-do 51480 (KR); KIM, Dong-ho, Changwon-si Gyeongsangnam-do 51382 (KR); PARK, Sung-gyu, Changwon-si Gyeongsangnam-do 51531 (KR)
(74) Representative: Kador & Partner Part mbB
(86) International application number: PCT/KR2022/020335
(87) International publication number: WO 2023/234506

(57) **Abstract**

The present disclosure provides a substrate for spectroscopic analysis comprising a polycrystalline structure and a preparation method thereof. More specifically, the present disclosure provides to a substrate for spectroscopic analysis based on universal materials comprising a polycrystalline structure with enhanced signal intensity and signal uniformity by comprising a polycrystalline structure, and a preparation method of a substrate for spectroscopic analysis comprising a polycrystalline structure whose shape is controlled by a simple process on surface of various materials.

## Description

### Technical Field

The present disclosure relates to a substrate for spectroscopic analysis comprising a polycrystalline structure and a preparation method thereof. More specifically, the present disclosure relates to a substrate for spectroscopic analysis based on universal materials comprising a polycrystalline structure with enhanced signal intensity and signal uniformity, and a preparation method of a substrate for spectroscopic analysis comprising a polycrystalline structure whose shape is controlled by a simple process on surface of various materials.

### Background

Raman scattering refers to an inelastic scattering through which energy of incident light changes and a phenomenon that, when incident light is applied on a specific molecule, light with wavelength slightly different from that of the incident light is produced by intrinsic property of vibrational transition of the molecule.

In fact, almost all organic molecules have their intrinsic Raman shift so that signals may be collected even from a nonpolar molecule with induced polarization change through Raman spectroscopy using Raman scattering. In addition, Raman spectroscopy is not interfered by water molecules, and therefore, it is more suitable for detections of biomolecules, such as proteins and genes.

A wavelength of a Raman emitted spectrum indicates characteristics of a chemical composition and structure of a light absorbing molecule within a sample. Therefore, a subject material of an analysis may be directly analyzed through analysis of such Raman signals.

As background of the present disclosure, a method for preparation of paper-based surface enhanced Raman scattering substrate using successive chemical reaction method is described in Korean Patent No. 10-1867670. However, in the above patent, not only the process is complex due to requiring preparation of metal nanoparticles having diameters of 1 to 100 nm and use of a first metal precursor solution, a second metal precursor solution, a first reducing agent solution, and a second reducing agent solution, but also there remains a problem that control of the process is not easy due to repeated actions of adsorbing a precursor, washing off a precursor, soaking in a reducing agent solution, and washing off a reducing agent solution for several times.

### Summary of The Invention

A purpose of the present disclosure is to provide a substrate for spectroscopic analysis with superior sensitivity and signal uniformity when in use for spectroscopic analysis, the substrate formed by direct growth of nanoparticles containing noble metal on a base member.

Another purpose of the present disclosure is to provide a substrate for spectroscopic analysis easily applicable on various 3-dimension surface with superior flexibility.

Another purpose of the present disclosure is to provide a composition for preparation of a substrate for spectroscopic analysis through which a shape of a noble metal nanocrystal particle formed regardless of a material of a base member may be easily controlled and polycrystalline particles may be easily formed, thereby enhancing signal sensitivity and signal uniformity of Surface Enhanced Raman Spectroscopy (SERS).

Another purpose of the present disclosure is to provide a Raman spectroscopic device capable of on-site diagnosis of a disease, such as cancer.

Another purpose of the present disclosure is to provide a preparation method of a substrate for spectroscopic analysis through which a substrate for spectroscopic analysis with high sensitivity and signal uniformity of SERS signal may be efficiently prepared by a simple process regardless of a base member material.

Purposes of the present disclosure are not limited to the purposes stated above. Other unstated purposes should be clearly understood from the detailed description.

According to one aspect, a substrate for spectroscopic analysis comprising a base member; and a polycrystalline structure composed of a cluster of a plurality of nanoparticles formed on the base member to have a plurality of grain boundaries, the substrate formed through a liquid process, is provided.

According to one embodiment, the base member may be composed of at least one species of a paper, a polymer, a well plate, a wafer, and a protein.

According to one embodiment, the nanoparticles may be interconnected in a branch structure, and the polycrystalline structure may have a diameter of 1µm to 100µm in average.

According to one embodiment, a substrate for spectroscopic analysis of the present disclosure may have an Electrochemically Active Surface Area (EASA) of 0.1 cm² to 10.0 cm².

According to one embodiment, the nanoparticle may be composed of at least one species of Au, Ag, and Pt.

According to one embodiment, the substrate for spectroscopic analysis may be applicable to a Raman spectroscopy system using a wavelength of at least 500 nm, or a near-IR FT Raman spectroscopy system using a wavelength of 1064nm.

According to one embodiment, the substrate for spectroscopic analysis may be composed of a 3-dimension swab stick, a wearable substrate, or a plasmonic well plate.

According to another aspect, a composition for preparing a substrate for spectroscopic analysis comprising a noble metal precursor and a reducing agent, wherein a ratio of the reducing agent to the noble precursor is 1:0.5 to 1:15, is provided.

According to one embodiment, as a ratio of the reducing agent to the noble metal precursor increases in a composition for preparing a substrate for spectroscopic analysis of the present disclosure, a size of the polycrystalline structure formed on a substrate for spectroscopic analysis may decrease and density of a polycrystalline structure increases present disclosure.

According to one embodiment, the noble metal precursor may be at least one species of HauCl₄ and NaAuCl₄.

According to one embodiment, the reducing agent may be at least one species of hydroxylamine, ascorbic acid, FeSO₄, and hydroxyquinone.

According to another aspect, a Raman spectroscopy device comprising a light source; a substrate for spectroscopic analysis according to the present disclosure; and a detector detecting Raman scattering is provided.

According to one embodiment, a Raman spectroscopy device of the present disclosure may further comprise an adaptor provided with a first end in an open cylindrical form, the first end provided with a joint unit fastening to the Raman spectroscopy device, and a second end, the second end provided with a coupling unit formed with a through hole inducing light from a light source; and a measurement unit in a spoon shape composed of a head unit and a straight body unit extended from the head unit, the head unit provided with a hemispherical seating unit to which the coupling unit of the second end of the adaptor is placed and a seating groove extended from the center of the body unit to the hemispherical seating unit in a longitudinal direction so that a straight strip substrate for spectroscopic analysis is placed.

According to one embodiment, the seating groove may be formed in a T-shape so that an end of the strip substrate for spectroscopic analysis does not deviate from the hemispherical seating unit.

According to one embodiment, a target material of the detector may be selected from at least one species of a cell, a metabolite, a protein, a nucleic acid, DNA, RNA, an enzyme, an organic molecule, a virus, an extracellular vesicle, a microvesicle, an exosome, and fat in urine, saliva, sweat or tear.

According to one embodiment, the Raman spectroscopy device may allow on-site diagnosis of cancer.

According to another aspect, in a preparation method of a substrate for spectroscopic analysis of the present disclosure, the preparation method of a substrate for spectroscopic analysis comprising a) preparing a base member for preparation of a base member; and b) forming a polycrystalline structure with a plurality of grain boundaries composed of a cluster of a plurality of nanoparticles on the base member by soaking the base member in a composition for preparing a substrate for spectroscopic analysis comprising a solution of a noble metal precursor and a reducing agent, so that the step b) comprises controlling shape of the polycrystalline structure by having a ratio of a reducing agent to a noble metal precursor solution of 1:0.5 to 1:15 , is provided.

According to one embodiment, the step a) in a preparation method of a substrate for spectroscopic analysis of the present disclosure may further comprise surface modifying for surface modification of the base member surface prior to formation of a polycrystalline structure.

According to one embodiment, surface modifying in a preparation method of a substrate for spectroscopic analysis of the present disclosure may comprise treating the base member surface with a surface modifying composition comprising at least one species selected from base of 1 to 3% and acid of 1 to 3%, and alcohol of C₁₋₅ or water as solvent.

### Effects of Invention

According to one embodiment, a substrate for spectroscopic analysis of the present disclosure has superior sensitivity and signal uniformity when it is used for spectroscopic analysis because nanoparticles having noble metal is directly grown to be interconnected in a branch structure and a microparticle size polycrystalline structure can be comprised.

According to one embodiment, a base member of a substrate for spectroscopic analysis of the present disclosure has superior flexibility and is easily applicable to various 3-dimension surfaces due to composition of various flexible materials.

According to one embodiment, a composition for preparing a substrate for spectroscopic analysis of the present disclosure may increase sensitivity and signal uniformity of SERS signals by controlling a ratio between a noble metal precursor and a reducing agent, thereby easily controlling a shape of a noble metal nanocrystal particle, which is formed regardless of a base member material, and by easily forming polycrystal particles.

According to one embodiment, a Raman spectroscopy device of the present disclosure may allow on-site verification of a type of a non-label subject material and on-site quantitative analysis.

According to one embodiment, a Raman spectroscopy device of the present disclosure allows on-site diagnosis of a disease, such as cancer, using a small amount of a biological specimen, such as urine.

According to one embodiment, a preparation method of a substrate for spectroscopic analysis of the present disclosure allows efficient preparation of a substrate for spectroscopic analysis with high SERS signal intensity and signal uniformity by direct growth of a polycrystalline structure including noble metal on a base member through a simple process referred as a one-time solution process regardless of a base member type.

According to one embodiment, a preparation method of a substrate for spectroscopic analysis of the present disclosure may allow efficient preparation of a substrate for spectroscopic analysis having high particle density and enhanced signal uniformity through surface modification.

### Brief Description of The Drawings

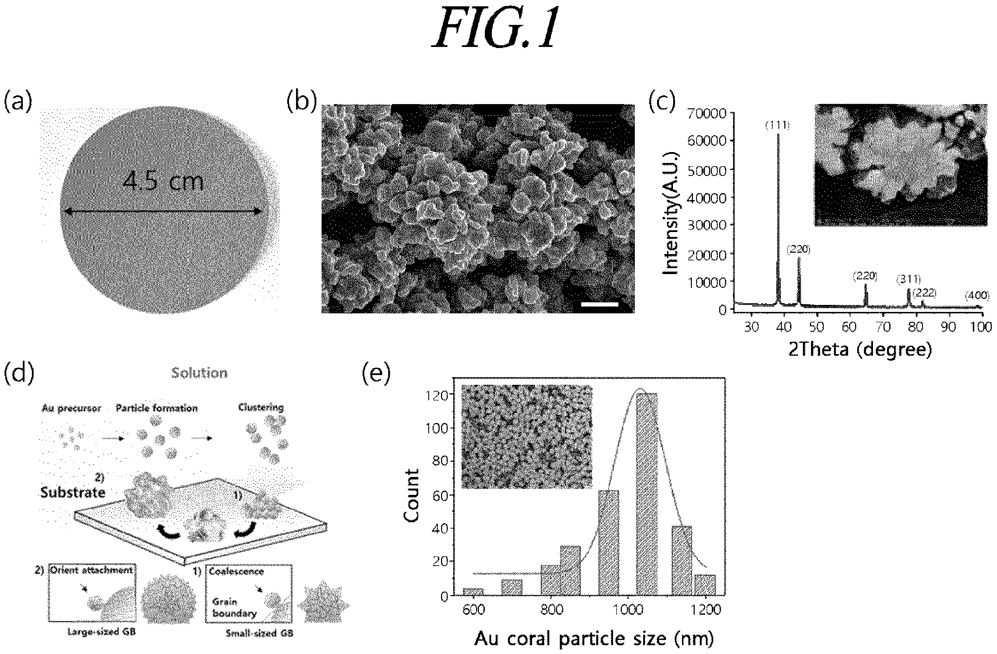

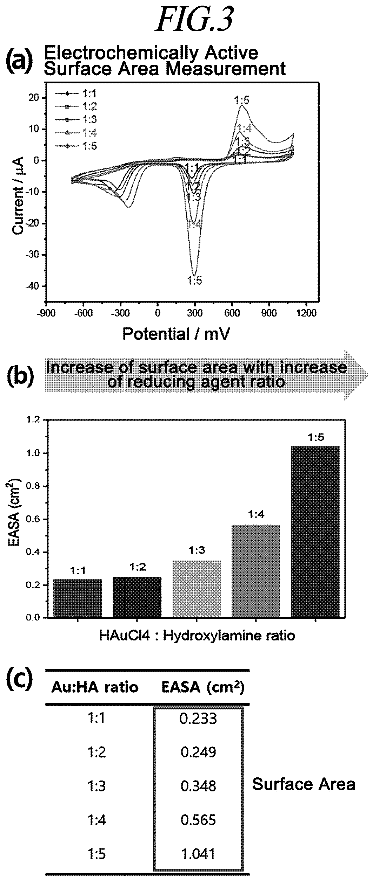

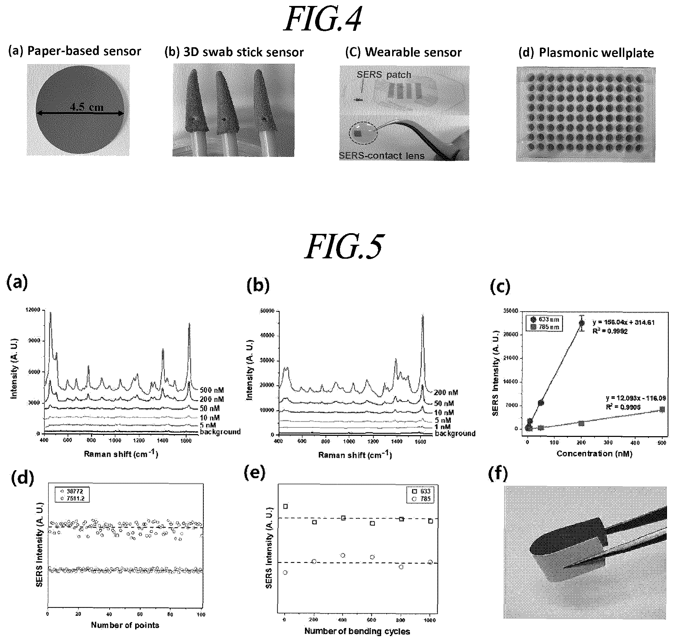

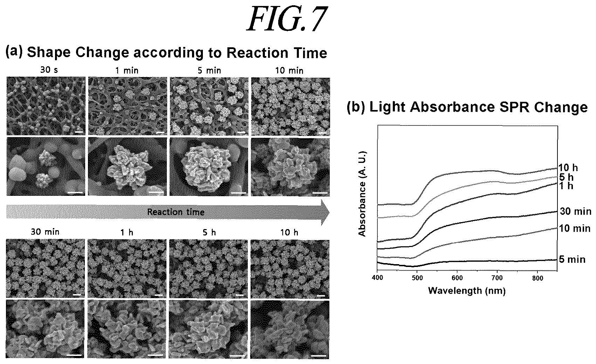

(a) in FIG. 1 illustrates a paper-based substrate for spectroscopic analysis with a base member of cellulose acetate according to one embodiment of the present disclosure. (b) to (e) in FIG.1 illustrate a SEM photograph ((b) in FIG. 1), a polycrystalline verification result ((c) in FIG.1), a particle formation mechanism ((d) in FIG. 1), and a particle distribution graph ((e) in FIG.1) of a substrate.

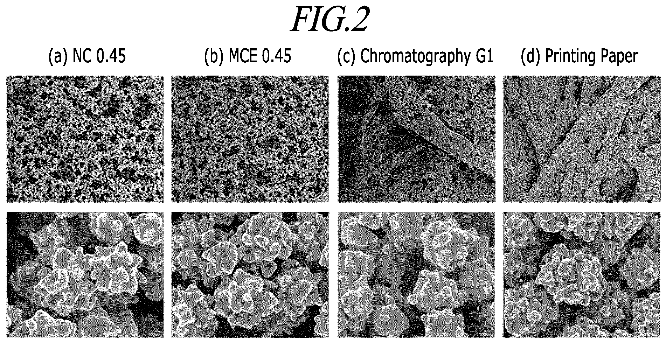

FIG. 2 is a SEM photograph illustrating a shape of a grown particle with base members of a nitro cellulose (NC), mixed cellulose ester (MCE), a chromatography paper, and a printing paper according to embodiments of the present disclosure.
(a) to (c) in FIG. 3 are diagrams illustrating results of measurements of electrochemical surface areas according to a reducing agent ratio included in a composition for preparing a substrate for spectroscopic analysis according to embodiments of the present disclosure.
(a) to (d) in FIG. 4 are photographs illustrating substrates for spectroscopic analysis in various forms according to embodiments of the present disclosure.
(a) to (c) in FIG. 5 are graphs illustrating SERS signal intensities at wavelengths of 633nm and 785nm by treating with different concentrations of methylene blue (MB) while the reducing agent applied to a substrate for spectroscopic analysis according to one embodiment of the present disclosure is hydroxylamine (a ratio of a reducing agent to a metal precursor is 1:2). (d) and (e) in FIG. 5 are graphs illustrating signal uniformity of a substrate for spectroscopic analysis according to one embodiment of the present disclosure, and (f) in FIG. 5 is a photograph illustrating flexibility of a substrate for spectroscopic analysis according to one embodiment of the present disclosure.

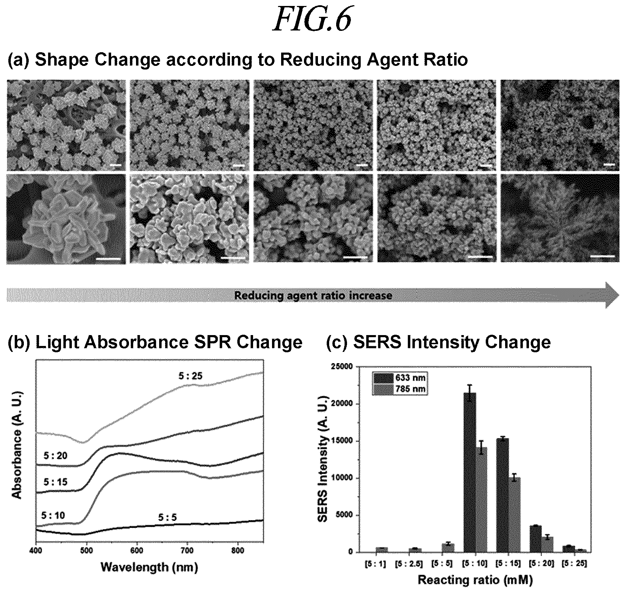

(a) in FIG. 6 is a SEM photograph illustrating a particle shape change according to a ratio between a metal precursor and a reducing agent in a composition for preparing a substrate for spectroscopic analysis according to one embodiment of the present disclosure. (b) in FIG. 6 illustrates light absorption surface plasmon resonance (SPR) change of a substrate for spectroscopic analysis per wavelength according to a ratio between a metal precursor and a reducing agent in a composition for preparing a substrate for spectroscopic analysis according to one embodiment of the present disclosure. (c) in FIG. 6 is a graph illustrating SERS signal intensity change of a substrate for spectroscopic analysis per wavelength according to a ratio between a metal ion precursor and a reducing agent in a composition for preparing a substrate for spectroscopic analysis according to one embodiment of the present disclosure.
(a) and (b) in FIG. 7 are a SEM photograph ((a) in FIG. 7) illustrating shape change of a particle according to a reaction time after soaking a base member in a composition for preparing a substrate for spectroscopic analysis according to the present disclosure and a graph ((b) in FIG. 7) illustrating corresponding light absorbance SPR change.

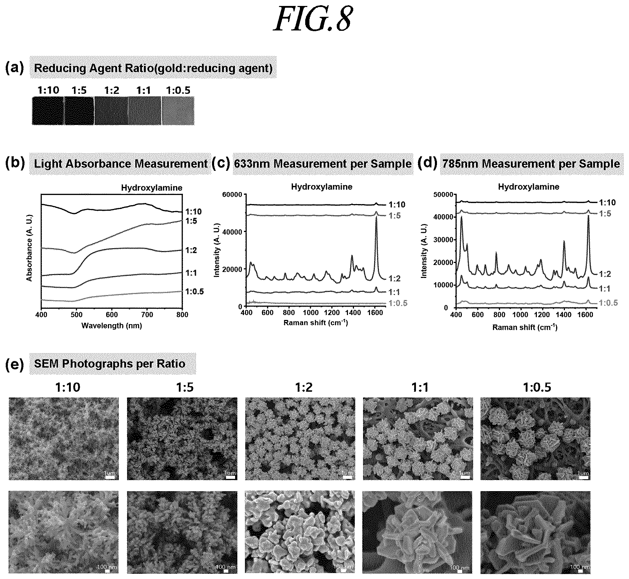

(a) in FIG. 8 is a photograph illustrating color change of a substrate for spectroscopic analysis according to a ratio between a metal ion precursor and a hydroxylamine reducing agent in a composition for preparing a substrate for spectroscopic analysis according to one embodiment of the present disclosure. (b) in FIG. 8 is a graph illustrating light absorbance SPR change of a substrate for spectroscopic analysis of (a) in FIG. 8. (c) in FIG. 8 is a graph illustrating SERS signal intensity change of a substrate for spectroscopic analysis of (a) in FIG. 8 measured at 633nm. (d) in FIG. 8 is a graph illustrating SERS signal intensity change of a substrate for spectroscopic analysis of (a) in FIG. 8 measured at 785 nm. (e) in FIG. 8 is a SEM photograph illustrating a particle shape of a substrate for spectroscopic analysis according to a ratio between a metal ion precursor and a hydroxylamine reducing agent in a composition for preparing a substrate for spectroscopic analysis according to one embodiment of the present disclosure.

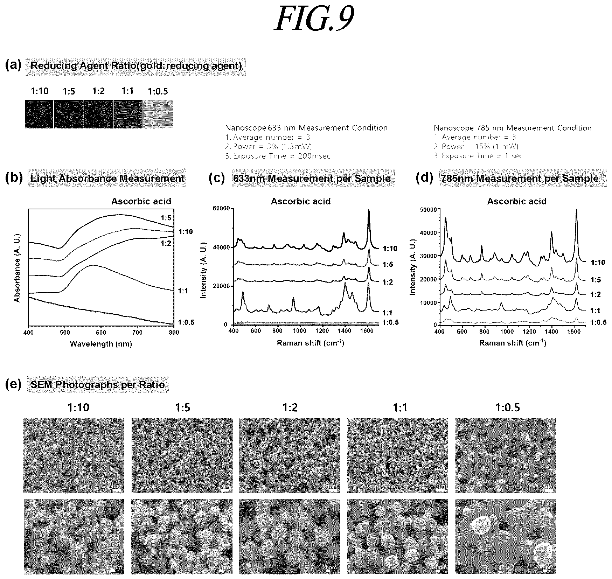

(a) in FIG. 9 is a photograph illustrating color change of a substrate for spectroscopic analysis according to a ratio between a metal ion precursor and an ascorbic reducing agent in a composition for preparing a substrate for spectroscopic analysis according to one embodiment of the present disclosure. (b) in FIG. 9 is a graph illustrating light absorbance SPR change of a substrate for spectroscopic analysis of (a) in FIG. 9. (c) in FIG. 9 is a graph illustrating SERS signal intensity change of measurement of a substrate for spectroscopic analysis of (a) in FIG. 9 at 633 nm. (d) in FIG. 9 is a graph illustrating SERS signal intensity change of a substrate for spectroscopic analysis of (a) in FIG. 9 measured at 785 nm. (e) in FIG. 9 is a SEM photograph illustrating a particle shape of a substrate for spectroscopic analysis according to a ratio of a metal ion precursor and an ascorbic acid reducing agent in a composition for preparing a substrate for spectroscopic analysis according to one embodiment of the present disclosure.

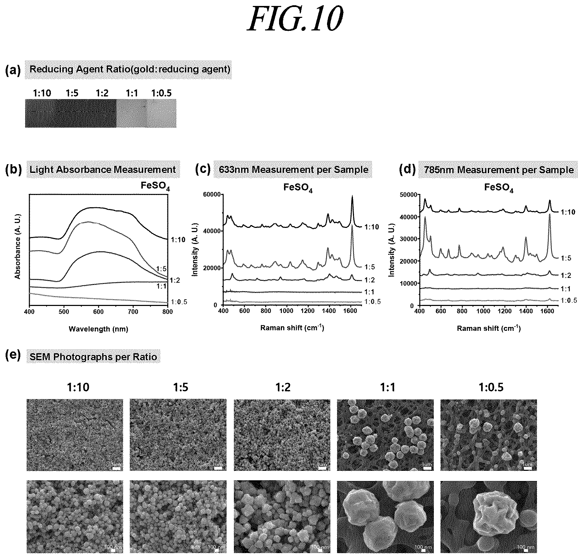

(a) in FIG. 10 is a photograph illustrating a color change of a substrate for spectroscopic analysis according to a ratio between a metal ion precursor and a FeSO₄ reducing agent in a composition for preparing a substrate for spectroscopic analysis according to one embodiment of the present disclosure. (b) in FIG. 10 is a graph illustrating light absorbance SPR change of a substrate for spectroscopic analysis of (a) in FIG. 10. is a graph illustrating SERS signal intensity(c) in FIG. 10 change of a substrate for spectroscopic analysis of (a) in FIG. 10 measured at 633 nm. (d) in FIG. 10 is a graph illustrating SERS signal intensity change of a substrate for spectroscopic analysis of (a) in FIG. 10 measured at 785nm. (e) in FIG. 10 is a SEM photograph illustrating a particle shape of a substrate for spectroscopic analysis according to a ratio between a metal ion precursor and a FeSO₄ reducing agent in a composition for preparing a substrate for spectroscopic analysis according to one embodiment of the present disclosure.

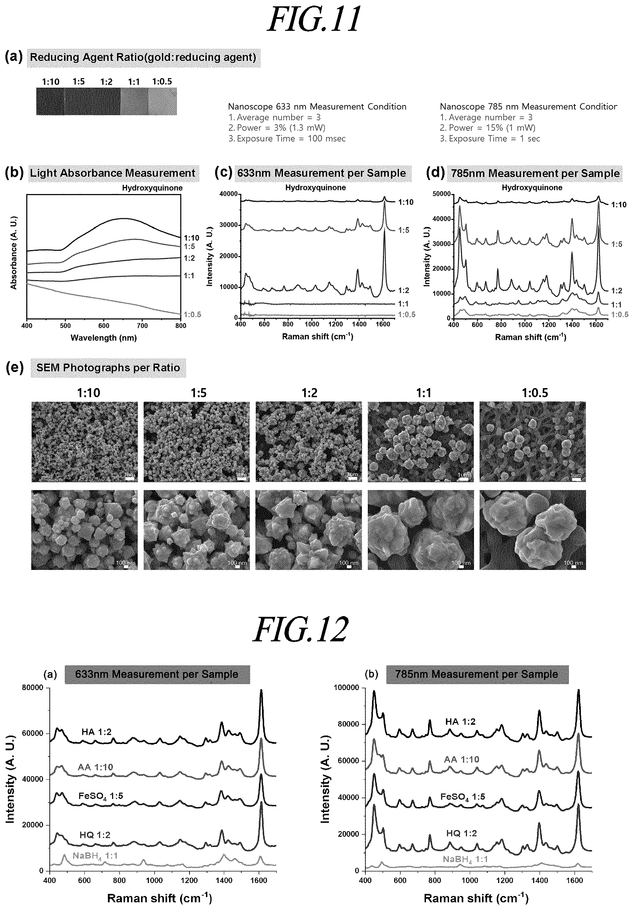

(a) in FIG. 11 is a photograph illustrating a color change of a substrate for spectroscopic analysis according to a ratio between a metal ion precursor and a hydroxyquinone reducing agent in a composition for preparing a substrate for spectroscopic analysis according to one embodiment of the present disclosure. (b) in FIG. 11 is a graph illustrating light absorbance SPR change of a substrate for spectroscopic analysis of (a) in FIG. 11. (c) in FIG. 11 is a graph illustrating SERS signal intensity change of a substrate for spectroscopic analysis of (a) in FIG. 11 measured at 633nm. (d) in FIG. 11 is a graph illustrating SERS signal intensity change of a substrate for spectroscopic analysis of (a) in FIG. 10 measured at 785nm. (e) in FIG. 11 is a SEM photograph illustrating a particle shape of a substrate for spectroscopic analysis according to a ratio between a metal ion precursor and a hydroxyquinone reducing agent in a composition for preparing a substrate for spectroscopic analysis according to one embodiment of the present disclosure.
(a) in FIG. 12 and (b) in illustrate comparisons of SERS intensities at wavelengths ofFIG. 12 633nm ((a) in FIG. 12) and 785nm ((b) in FIG. 12) of a substrate for spectroscopic analysis prepared with a composition for preparing a substrate for spectroscopic analysis having an optimum ratio of a metal ion precursor and a respective reducing agent according to embodiments of the present disclosure.

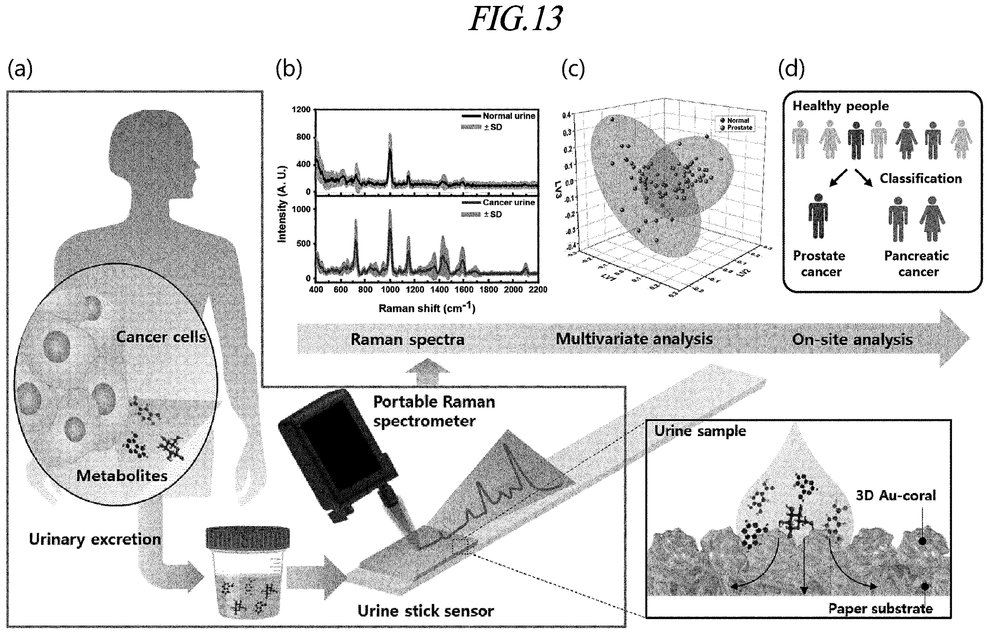

(a) in FIG. 13 to (d) in FIG. 13 schematically illustrate a method for on-site diagnosis of cancer using a portable Raman spectroscopy device ((a) in FIG. 13) according to one embodiment of the present disclosure.

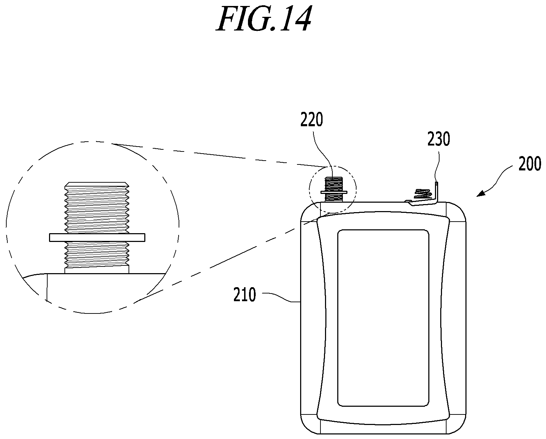

FIG. 14 is a diagram illustrating a Raman spectroscopy device according to one embodiment of the present disclosure.

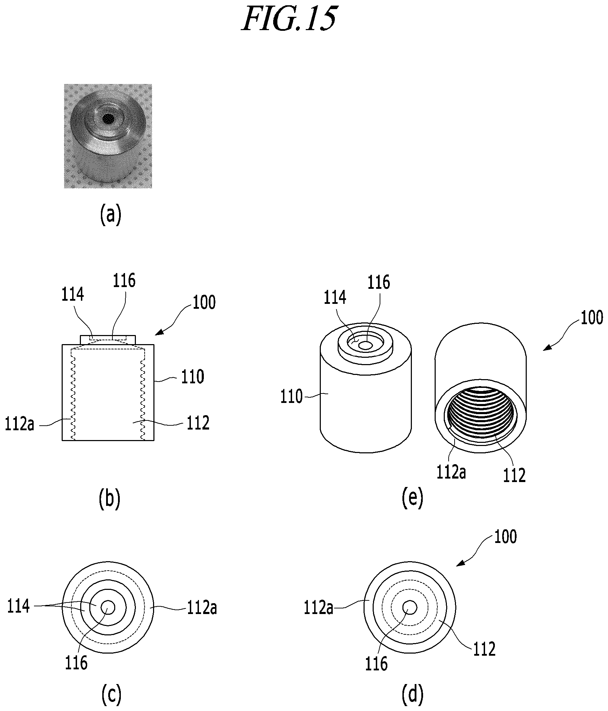

(a) in FIG. 15 is a photograph of a perspective view of an adaptor for a Raman spectroscopy device according to one embodiment of the present disclosure. (b) in FIG. 15 is a front view of an adaptor of (a) in FIG. 15. (c) of FIG. 15 is a plane view of an adaptor of (a) in FIG. 15. (d) in FIG. 15 is a bottom view of an adaptor of (a) in FIG. 15. (e) in FIG. 15 is a perspective view of an adaptor of (a) in FIG. 15.

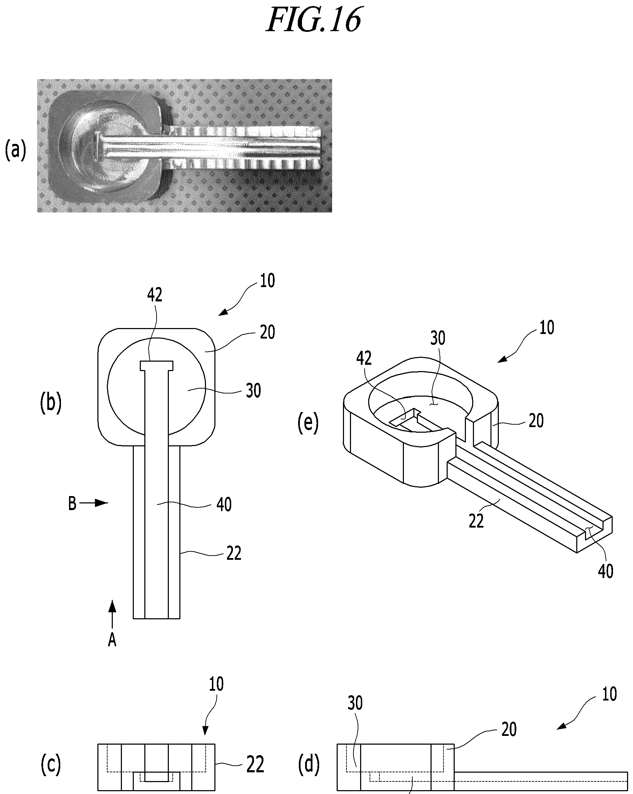

(a) in FIG. 16 is a photograph of a plane view of an analysis member for a Raman spectroscopy device according to one embodiment of the present disclosure. (b) in FIG. 16 is a plane view of an analysis member of (a) in FIG. 16. (c) in FIG. 16 is a front view in a direction of A of an analysis member of (a) in FIG. 16. (d) in FIG. 16 is a side view in a direction of B of an analysis member of (a) in FIG. 16. (e) in FIG. 16 is a perspective view of an analysis member of (a) in FIG. 16.

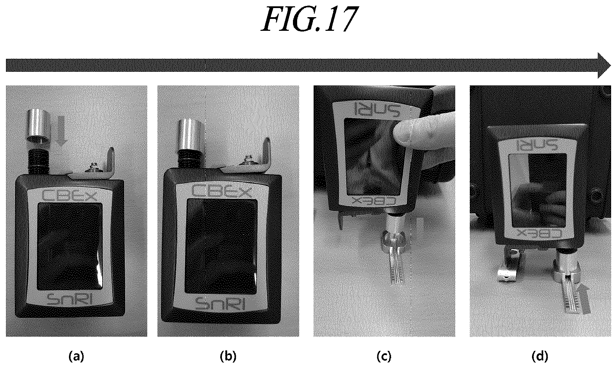

FIG. 17 is a photograph schematically illustrating an analysis method by coupling an adaptor and an analysis member with a Raman spectroscopy device according to one embodiment.

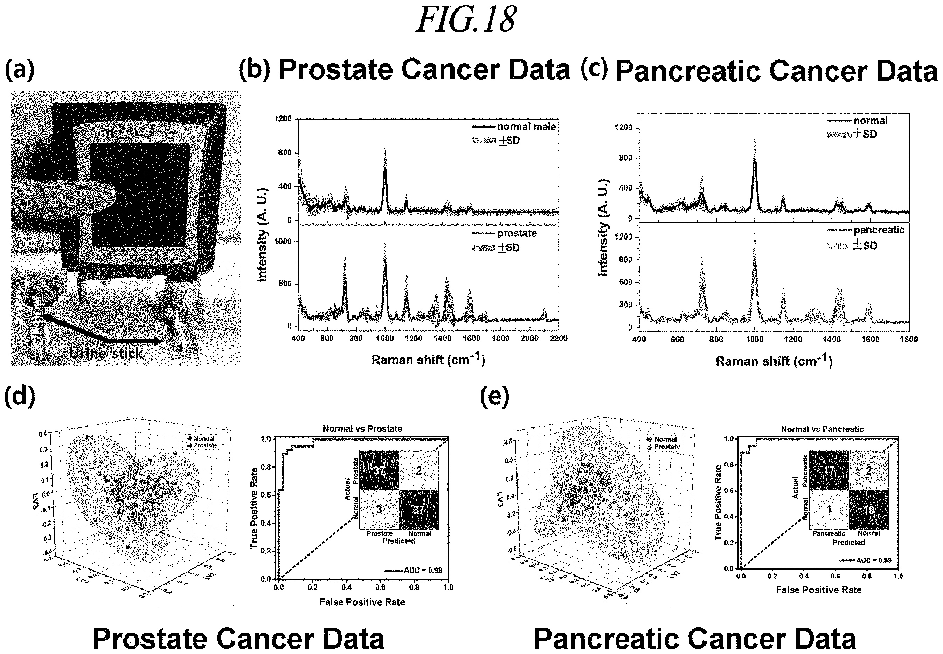

FIG. 18 illustrates Raman spectra of prostate cancer and pancreatic cancer (see (b) in FIG. 18) and (c) in FIG. 18) measured by a Raman spectroscopy device having a substrate for spectroscopic analysis according to one embodiment of the present disclosure (see (a) in FIG. 18) and a multivariate analysis data of prostate cancer and pancreatic cancer based thereof (see (d) and (e) in FIG. 18).

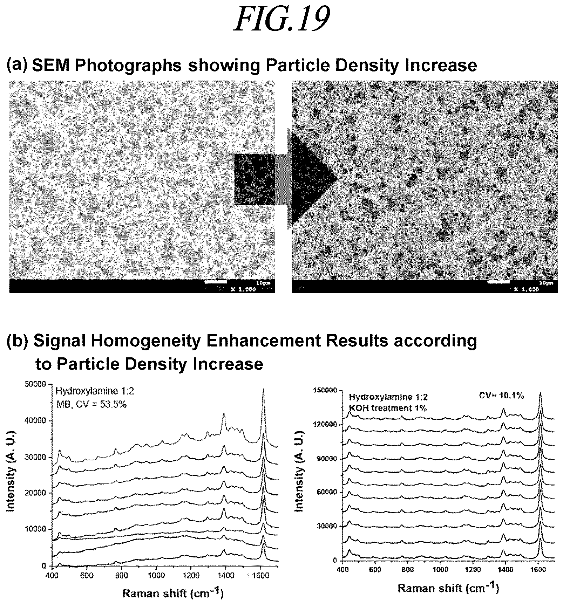

(a) in FIG. 19 is a SEM photograph illustrating a result of increased particle density through surface modification using a surface modifying composition according to one embodiment of the present disclosure, and (b) in FIG. 19 is a graph illustrating a result of enhanced signal uniformity accordingly.

### Detailed Description

Purposes, specific advantages, and noble characteristics of the present disclosure will become much clearer through the detailed description and embodiments, hereinafter, in relation to the attached figures.

First, terms or words used in this specification and claims should not be construed as their general dictionary meanings, but as a meaning and a concept that correspond to the technical idea of the present disclosure based on the principle that an inventor may appropriately define a concept of a term in order to explain his/her invention to the best method.

In the specification, when elements, such as a layer, a part, or a substrate, are described to be "on," "connected to," or "coupled to" another element, such descriptions may refer that the elements are directly "on," "connected to," or "coupled to" the another element, or that at least another element may be interposed between the two elements. In the contrary, when an element is described to be "directly on," "directly connected to," or "directly coupled to" another element, there is no other element interposed between the two elements.

Terms used in the specification are merely used for explanation of specific embodiments and are not intended to limit the present disclosure. Singular expressions include plural expressions unless the context clearly indicates otherwise.

Terms, such as "comprise" or "have," described in the specification are merely for indicating presence of characteristics, numbers, steps, actions, elements, parts or combinations thereof. The terms should not be interpreted as preliminarily excluding possibilities of presence or addition of at least one other characteristic, number, action, element, part, or combination thereof.

In the specification, a description that a part "comprises" an element refers that, unless there is a specific contrary description, the element may be further comprised, and other elements are not excluded. Also, in the entire specification, the description of being "on" an object refers to a state of being disposed above or below a part of the object and does not necessarily mean the state of being above based on the gravitational direction.

The present disclosure may be applied with various variations and have several embodiments. Accordingly, specific examples are illustrated in figures and explained in detail in the detailed descriptions. Nonetheless, these are not to limit the present disclosure to specific embodiments. Instead, these should be understood that the disclosure includes every variation, and equivalent to substituent included in the scope of ideas and technology of the present disclosure. In explanation of the present disclosure, when detailed descriptions about a related, known technology may obscure the gist of the present disclosure, the detailed descriptions thereof are omitted.

Hereinafter, embodiments of the present disclosure are explained in detail with reference to the attached figures. In explanation with reference to the attached figures, the same or corresponding components shall be assigned with the same reference figure numeral, and redundant descriptions thereof shall be omitted.

(a) in FIG. 1 illustrates a paper-based substrate for spectroscopic analysis with a base member of cellulose acetate according to one embodiment of the present disclosure. (b) to (e) in FIG.1 illustrate a SEM photograph ((b) in FIG. 1), a polycrystalline verification result ((c) in FIG. 1), a particle formation mechanism ((d) in FIG. 1), and a particle distribution graph ((e) in FIG. 1) of a substrate. FIG. 2 is a SEM photograph illustrating a shape of a grown particle with base members of a nano cellulose (NC), mixed cellulose ester (MCE), a chromatography paper, and a printing paper according to embodiments of the present disclosure.

(a) to (c) in FIG. 3 are diagrams illustrating results of measurements of electrochemical surface areas by a reducing agent ratio included in a composition for preparing a substrate for spectroscopic analysis according to embodiments of the present disclosure.

A substrate for spectroscopic analysis of the present disclosure comprises a base member; and a polycrystalline structure composed of a cluster of a plurality of nanoparticles formed on the base member to have a plurality of grain boundaries and is formed by a solution process.

By referring to (a) to (e) in FIG. 1 and FIG. 2, formation of a polycrystalline with a cluster of a plurality of nanoparticles formed on cellulose acetate (see (a) to (e) in FIG. 1) and other various base members may be verified (see FIG. 2). By referring to (d) in FIG. 1, although not limited thereto, it is shown to compose a cluster, which, specifically, is directly grown on a base member by using a solution process, by formation of nanoparticles with an Au precursor among noble metals. Specifically, clustered nanoparticles form polygon structure grain of a small size by coalescence. Then, the grain gradually grows larger by oriented attachment growth. Accordingly, a polycrystalline structure having a plurality of grain boundaries is formed. Although not limited thereto, a particle in a noble metal coral shape, which is a polycrystalline structure, may grow to a micro size in average. (See (e) in FIG. 1) When used as a substrate for spectroscopic analysis, a polycrystalline structure having a plurality of grain boundaries as above may dramatically enhance signal intensity and signal uniformity as scattering increases at a plurality of grain boundaries.

A base member applicable to the present disclosure does not have specific requirement, and various materials may be used. Although not limited thereto, the base member may be composed of at least one species of a paper, a polymer, a well plate, a wafer, and a protein. Although not limited thereto, the paper may be papers such as cellulose acetate, nitro cellulose (NC), mixed cellulose ester (MCE), a chromatography paper and a commonly used printing paper. In addition, a substrate for spectroscopic analysis may be a porous polymer, a plasmonic well plate, a wafer or a protein film composed of protein of at least one species of silk, collagen, chitin, elastin, and keratin. Referring to FIG. 2, a form of a polycrystalline structure may be shown in a similar structure formed on a base member based on nitro cellulose (NC) (see (a) in FIG. 2), mixed cellulose ester (MCE) ((b) in FIG.2), a chromatography paper ((c) in FIG.2), and a commonly used printing paper ((d) in FIG. 2). Although not limited thereto, depending on the applied field, a base member provided with porosity and/or flexibility may be suitable, and a paper, a polymer or a protein film may be suitable. In addition, a plasmonic well plate may be suitable for simultaneous detection of a plurality of specimens.

Although not limited thereto, the nanoparticles may be interconnected in a branch form, and the polycrystalline structure may have an average diameter of 1µm to 100µm. A substrate for spectroscopic analysis of the present disclosure may grow into a polycrystalline structure through a solution process while it may be interconnected in a branch form. The grown polycrystalline structure may have an average diameter of 1µm to 100µm. Although not limited thereto, the average diameter of the polycrystalline structure may be suitable for enhancement of signal intensity and signal uniformity within the said range.

Although not limited thereto, a substrate for spectroscopic analysis may have an electrochemically active surface area (EASA) of 0.1 cm² of to 10.0 cm². A substrate for spectroscopic analysis of the present disclosure may grow into a polycrystalline structure through a solution process so that its surface area may increase. In addition, by referring to (a) to (c) in FIG. 3, it can be shown that the electrochemically active surface area (EASA, cm²) increases as a ratio of a reducing agent to a metal ion precursor included in a composition for preparing a substrate for spectroscopic analysis according to the present disclosure increases. Meanwhile, electricity may not flow due to low density of a noble metal polycrystalline structure within the range of a ratio of a reducing agent to a metal ion precursor less than 1:0.5.

Although not limited thereto, the nanoparticle may be composed of at least one species of Au, Ag, and Pt. Au may be the most suitable for enhancing signal intensity and signal uniformity.

Although not limited thereto, a substrate for spectroscopic analysis of the present disclosure may be applicable to a Raman spectroscopy system using wavelength of at least 500nm or a near-IR FT Raman spectroscopy system using a wavelength of 1064 nm.

Referring to (b) in FIG. 6, a light absorbance range is shown to be as broad as a range of wavelengths of 500 nm or higher when a substrate for spectroscopic analysis according to the present disclosure is used. Accordingly, it can be verified that a variety of wavelengths of current use may be applied to the Raman spectroscopy system. Although not limited thereto, it may be more suitable at wavelengths of 633nm or 785nm.

(a) to (d) in FIG. 4 are photographs illustrating substrates for spectroscopic analysis in various forms according to embodiments of the present disclosure.

Although not limited thereto, the substrate for spectroscopic analysis may be composed of a 3-dimension swab stick, a wearable substrate, or a plasmonic well plate. A substrate for spectroscopic analysis of the present disclosure may comprise various base members so that it may be prepared with flexible material and have other shapes. Referring to FIG. 4, various forms, such as a 3D swab stick, a wearable substrate form, a well plate form, a protein film form, that use 3-dimension porous materials, other than a plane shaped substrate, may be applicable.

The substrate for spectroscopic analysis in a 3-dimension swab stick form may be useful for collection of a sample, the wearable substrate allows collection of comparatively long-time collection of a specimen due to a composition in a form of a patch or a lens, which allows collection of a specimen from an eye. In addition, a plasmonic well plate may shorten the analysis time as multiple specimens can be analyzed simultaneously.

According to another aspect of the present disclosure, a composition for preparing a substrate for spectroscopic analysis of the present disclosure comprises a noble metal precursor and a reducing agent.

(a) to (c) in FIG. 5 are graphs illustrating SERS signal intensities at 633nm and 785nm by applying different concentrations of methylene blue (MB) while the reducing agent applied to a substrate for spectroscopic analysis according to one embodiment of the present disclosure is hydroxylamine (a ratio of a reducing agent to a metal precursor is 1:2). (d) and (e) in FIG. 5 are graphs illustrating signal uniformity of a substrate for spectroscopic analysis according to one embodiment of the present disclosure, and (f) in FIG. 5 is a photograph illustrating flexibility of a substrate for spectroscopic analysis according to one embodiment of the present disclosure.

Referring to (a) to (f) in FIG. 5, by using a substrate for spectroscopic analysis of the present disclosure prepared with base member of cellulose acetate, SERS signal intensity was measured by concentration of methylene blue (MB), and then SERS signal intensity was demonstrated at 633nm and 785nm, respectively, per methylene blue concentration. When a substrate for spectroscopic analysis of the present disclosure is used, it can be demonstrated that uniformity of a signal is high and consistent. In addition, a substrate for spectroscopic analysis of the present disclosure is small while it has flexibility. Therefore, it is easy to carry and applicable to various surfaces.

Although not limited thereto, when a ratio of a reducing agent to a noble metal precursor is less than 1:0.5, a Raman spectroscopy system of various wavelengths cannot be used. When the ratio is over 1:15, a substrate for spectroscopic analysis with superior signal intensity and signal uniformity is difficult to be obtained due to filmization.

Accordingly, a ratio of a reducing agent to a noble metal precursor in a composition for preparing a substrate for spectroscopic analysis of the present disclosure in the range of 1:0.5 to 1:15 may be suitable for preparation of a substrate for spectroscopic analysis with superior signal intensity and signal uniformity. The ratio may be from 1:0.5 to 1:12, 1:0.5 to 1:10, 1:1 to 1:10, 1:1 to 1:9, 1:1 to 1:8, 1:1 to 1:1.7, 1:1 to 1.6, 1:1 to 1:5, 1:1 to 1:4, or 1:1 to 1:3. In addition, the range may be slightly different depending on the type of a reducing agent.

Although not limited thereto, the greater a ratio of a reducing agent to a noble precursor increases in a composition for preparing a substrate for spectroscopic analysis of the present disclosure, the more a size of a polycrystalline structure formed on a substrate for spectroscopic analysis decreases and the more density of the polycrystalline structure increases present disclosure.

(a) in FIG. 6 is a SEM photograph illustrating a particle shape change according to a ratio between a metal precursor and a reducing agent in a composition for preparing a substrate for spectroscopic analysis according to one embodiment of the present disclosure. (b) in FIG. 6 illustrates surface light absorption plasmon resonance (SPR) change of a substrate for spectroscopic analysis per wavelength according to a ratio between a metal ion precursor and a reducing agent in a composition for preparing a substrate for spectroscopic analysis according to one embodiment of the present disclosure. (c) in FIG. 6 is a graph illustrating SERS signal intensity change of a substrate for spectroscopic analysis per wavelength according to a ratio between a metal ion precursor and a reducing agent in a composition for preparing a substrate for spectroscopic analysis according to one embodiment of the present disclosure.

Referring to FIG. 6, when a ratio of a reducing agent to a metal precursor is low, a size of a polycrystalline structure is large, and number of interconnections in a branch form is small. However, it can be demonstrated that, as a ratio of a reducing agent to a metal precursor increases, a size of a polycrystalline structure becomes small and interconnected in a greater number of branches. Meanwhile, depending on an optimum range of ratios of a reducing agent to a metal precursor in a composition for preparation of a substrate for spectroscopic analysis, density of a polycrystalline structure increases so that transmittance decreases and light absorbance increases. Accordingly, a Raman spectroscopy device with the higher sensitivity may be prepared.

(a) and (b) in FIG. 7 are a SEM photograph ((a) in FIG. 7) illustrating shape change of a particle according to a reaction time after soaking a base member in a composition for preparing a substrate for spectroscopic analysis according to the present disclosure and a graph ((b) in FIG. 7) illustrating corresponding light absorbance SPR change.

In addition, referring to FIG. 7, shapes of particles are shown according to time for soaking a substrate in a composition for preparing a substrate for spectroscopic analysis according to the present disclosure. Specifically, it can be demonstrated that, as the time for soaking increases, light absorbance increases.

Although not limited thereto, the noble metal precursor may be selected from a group composed of HAuCl₄, AuCl, AuCl₂, AuCl₃, Na₂Au₂Cl₈ and NaAuCl₂. However, at least one species of HAuCl₄ and NaAuCl₄ may be more suitable.

Although not limited thereto, the reducing agent may be at least one species of hydroxylamine, ascorbic acid, FeSO₄, and hydroxyquinone.

(a) in FIG. 8 to FIG. 12 show optimum ratios of a reducing agent to a noble metal precursor through comparisons of SERS signal intensities when the used reducing agents are hydroxylamine, ascorbic acid, FeSO₄, and hydroxyquinone.

(a) in FIG. 8 is a photograph illustrating color change of a substrate for spectroscopic analysis according to a ratio between a metal ion precursor and a hydroxylamine reducing agent in a composition for preparing a substrate for spectroscopic analysis according to one embodiment of the present disclosure. (b) in FIG. 8 is a graph illustrating light absorbance SPR change of a substrate for spectroscopic analysis of (a) in FIG. 8. (c) in FIG. 8 is a graph illustrating SERS signal intensity change of a substrate for spectroscopic analysis of (a) in FIG. 8 measured at 633nm. (d) in FIG. 8 is a graph illustrating SERS signal intensity change of a substrate for spectroscopic analysis of (a) in FIG. 8 measured at 785 nm. (e) in FIG. 8 is a SEM photograph illustrating a particle shape of a substrate for spectroscopic analysis according to a ratio between a metal ion precursor and a hydroxylamine reducing agent in a composition for preparing a substrate for spectroscopic analysis according to one embodiment of the present disclosure.

Referring to FIG. 8, when the reducing agent is hydroxylamine, a particle density increases from a ratio of 1:1. As a ratio of a reducing agent to a noble metal precursor increased, SERS intensity increased. The optimum ratio may be set as a ratio of 1:2, which is observed with the most intense SERS signal, and the optimum ratio range may be set as at least 1:1 to less than 1:5.

(a) in FIG. 9 is a photograph illustrating color change of a substrate for spectroscopic analysis according to a ratio between a metal ion precursor and an ascorbic reducing agent in a composition for preparing a substrate for spectroscopic analysis according to one embodiment of the present disclosure. (b) in FIG. 9 is a graph illustrating light absorbance SPR change of a substrate for spectroscopic analysis of (a) in FIG. 9. (c) in FIG. 9 is a graph illustrating SERS signal intensity change of a substrate for spectroscopic analysis of (a) in FIG. 9 measured at 633 nm. (d) in FIG. 9 is a graph illustrating SERS signal intensity change of a substrate for spectroscopic analysis of (a) in FIG. 9 measured at 785 nm. (e) in FIG. 9 is a SEM photograph illustrating a particle shape of a substrate for spectroscopic analysis according to a ratio of a metal ion precursor and an ascorbic acid reducing agent in a composition for preparing a substrate for spectroscopic analysis according to one embodiment of the present disclosure.

Referring to FIG. 9, when the reducing agent was ascorbic acid, SERS signal intensity at 785nm increased with the increase of a ratio of a reducing agent to a noble metal precursor. The optimum ratio may be set as 1:10, which is observed with the most intense SERS signal, and the optimum ratio range may be set as 1:0.5 to 1:15. Meanwhile, in the case of 633nm, the optimum ratio may be set as 1:1, which is observed with the most intense SERS signal, and the optimum ratio range may be set as 1:0.5 to 1:15.

(a) in FIG. 10 is a photograph illustrating a color change of a substrate for spectroscopic analysis according to a ratio between a metal ion precursor and a FeSO₄ reducing agent in a composition for preparing a substrate for spectroscopic analysis according to one embodiment of the present disclosure. (b) in FIG. 10 is a graph illustrating light absorbance SPR change of a substrate for spectroscopic analysis of (a) in FIG. 10. is a graph illustrating SERS signal intensity(c) in FIG. 10 change of a substrate for spectroscopic analysis of (a) in FIG. 10 measured at 633 nm. (d) in FIG. 10 is a graph illustrating SERS signal intensity change of a substrate for spectroscopic analysis of (a) in FIG. 10 measured at 785nm. (e) in FIG. 10 is a SEM photograph illustrating a particle shape of a substrate for spectroscopic analysis according to a ratio between a metal ion precursor and a FeSO₄ reducing agent in a composition for preparing a substrate for spectroscopic analysis according to one embodiment of the present disclosure.

Referring to FIG. 10, when the reducing agent was FeSO₄, SERS intensity increased with the increase in a ratio of a reducing agent to a noble metal precursor. The optimum ratio may be set as 1:5, which is observed with the most intense SERS signal, and the optimum ratio range may be set as more than 1:1 and at most 1:15.

(a) in FIG. 11 is a photograph illustrating a color change of a substrate for spectroscopic analysis according to a ratio between a metal ion precursor and a hydroxyquinone reducing agent in a composition for preparing a substrate for spectroscopic analysis according to one embodiment of the present disclosure. (b) in FIG. 11 is a graph illustrating light absorbance SPR change of a substrate for spectroscopic analysis of (a) in FIG. 11. (c) in FIG. 11 is a graph illustrating SERS signal intensity change of a substrate for spectroscopic analysis of (a) in FIG. 11 measured at 633nm. (d) in FIG. 11 is a graph illustrating SERS signal intensity change of a substrate for spectroscopic analysis of (a) in FIG. 11 measured at 785nm. (e) in FIG. 11 is a SEM photograph illustrating a particle shape of a substrate for spectroscopic analysis according to a ratio between a metal ion precursor and a hydroxyquinone reducing agent in a composition for preparing a substrate for spectroscopic analysis according to one embodiment of the present disclosure.

Referring to FIG. 11, when the reducing agent is hydroxyquinone, an optimum ratio may be set as 1:2, which is observed with the most intense SERS signal, and the optimum ratio range may be set as 1:0.5 to less than 1:15.

(a) in FIG. 12 and (b) in FIG. 12 illustrate comparisons of SERS intensities at wavelengths of 633nm ((a) in FIG. 12) and 785nm ((b) in FIG. 12) of a substrate for spectroscopic analysis prepared with a composition for preparing a substrate for spectroscopic analysis having an optimum ratio of a metal ion precursor and a respective reducing agent according to embodiments of the present disclosure.

Referring to FIG. 12, the optimum ratio, respectively for each reducing agent, was obtained while the optimization conditions for all reducing agents were obtained at wavelengths of 633nm and 785nm.

According to another aspect of the present disclosure, a Raman spectroscopy device comprising a light source; a substrate for spectroscopic analysis described in the present disclosure; and a detector detecting Raman spectra is provided.

(a) in FIG. 13 to (d) in FIG. 13 schematically illustrate a method for on-site diagnosis of cancer using a portable Raman spectroscopy device ((a) in FIG. 13) according to one embodiment of the present disclosure.

Referring to (a) in FIG. 13 to (d) in FIG. 13, a Raman spectroscopy device and an analysis method are schematically illustrated that Raman spectra were obtained from urinary excretion having metabolites of cancer cells by using a substrate for spectroscopic analysis (a sensor) and a portable Raman spectroscopy device according to the present disclosure and prostate cancer and pancreatic cancer are diagnosed through multivariate analysis and on-site analysis.

FIG. 14 is a diagram illustrating a Raman spectroscopy device according to one embodiment of the present disclosure. (a) in FIG. 15 is a photograph of a perspective view of an adaptor for a Raman spectroscopy device according to one embodiment of the present disclosure. (b) in FIG. 15 is a front view of an adaptor of (a) in FIG. 15. is a plane view of an adaptor of (a) in (c) of FIG. 15FIG. 15. (d) in FIG. 15 is a bottom view of an adaptor of (a) in FIG. 15. (e) in FIG. 15 is a perspective view of an adaptor of (a) in FIG. 15.

(a) in FIG. 16 is a photograph of a plane view of an analysis member for a Raman spectroscopy device according to one embodiment of the present disclosure. (b) in FIG. 16 is a plane view of an analysis member of (a) in FIG. 16. (c)4 in FIG. 16 is a front view in a direction of A of an analysis member of (a) in FIG. 16. (d) in FIG. 16 is a side view in a direction of B of an analysis member of (a) in FIG. 16. (e) in FIG. 16 is a perspective view of an analysis member of (a) in FIG. 16.

Referring to FIGS. 14 to 16, a Raman spectroscopy device 200 of the present disclosure may further comprise an adaptor 100 for coupling with the Raman spectroscopy device 200 and an analysis member 10 for placement and analysis of the adaptor 100 and a strip substrate for spectroscopic analysis.

Referring to FIGS. 14 to 16, the adaptor 100 is provided with a cylindrical body unit 110 having a hollow 112 with an open first end, and the first end is provided with a joint unit 112a for fastening with the Raman spectroscopy device 200. The joint unit 112a may be a screw thread formed on a side of the cylindrical body unit 110. In addition, a second end of the adaptor 100 is provided with a coupling unit 114 with a penetrating hole 116 for inducing light from a light source. The coupling unit 114 may be composed in a form for stable coupling with the analysis member 10.

Referring to FIGS. 15 and 16, the analysis member 10 is composed in a spoon shape composed of a head unit 20 and a straight body unit 22 extended from the head unit 20. The head unit 20 is provided with a hemispherical seating unit 30 for placement of the coupling unit 114 of the second end of the adaptor 100 and a seating groove 40 for placement of a straight strip substrate for spectroscopic analysis extended from the center of the body unit 22 to the hemispherical seating unit 30 in a longitudinal direction.

Although not limited thereto, the seating groove 40 may be provided with an end unit 42 extended from the seating unit 30 so that it is formed in a T-shape. Accordingly, the seating groove 40 may be characterized in that analysis accuracy may be increased by preventing the end of the strip substrate for spectroscopic analysis from deviating from the hemispherical seating unit 30.

FIG. 17 is a photograph schematically illustrating an analysis method by coupling an adaptor and an analysis member with a Raman spectroscopy device according to one embodiment.

Referring to FIG. 17, an adaptor is initially screwed to a Raman spectroscopy device. (See (a) and (b) in FIG. 17) Then, the adaptor fastened to a Raman spectroscopy device is coupled with an analysis member. (See (c) in FIG. 17) After inserting a strip substrate for spectroscopic analysis into the seating groove of the analysis member, an analysis is proceeded.

Although not limited thereto, a subject material of the detector may be selected from at least one species of a cell, a metabolite, a protein, a nucleic acid, DNA, RNA, an enzyme, an organic molecule, a virus, an extracellular vesicle, a microvesicle, an exosome and fat in urine, saliva, sweat or tear.

Although not limited thereto, the target material is a liquid specimen. Sensitivity has been very low for a liquid specimen due to low hygroscopicity of a conventional substrate for spectroscopic analysis. However, a substrate for spectroscopic analysis according to the present disclosure is hygroscopic, which allows quick on-site diagnosis even with a liquid specimen.

Although not limited thereto, the Raman spectroscopy analysis allows on-site diagnosis of cancer. Although not limited thereto, analysis of metabolites of the described target materials provides necessary information for diagnosis of cancer. Cancer may be diagnosed early through a non-invasive examination, which allows simultaneous analyses with extremely high level of sensitivity.

The cancer may be selected from at least one species composed of lung cancer, bronchial cancer, colon rectal cancer, prostate cancer, breast cancer, stomach cancer, ovarian cancer, bladder cancer, brain cancer, thyroid cancer, esophageal cancer, uterine cancer, liver cancer, kidney cancer, biliary cancer, prostate cancer, and pancreatic cancer. Although not limited thereto, it may be suitable for prostate cancer or pancreatic cancer.

FIG. 18 illustrates Raman spectra of prostate cancer and pancreatic cancer (see (b) in FIG. 18) and (c) in FIG. 18) measured by a Raman spectroscopy device having a substrate for spectroscopic analysis according to one embodiment of the present disclosure (see (a) in FIG. 18) and a multivariate analysis data of prostate cancer and pancreatic cancer based thereof (see (d) and (e) in FIG. 18).

According to another aspect, diagnosis of cancer using a Raman spectroscopy device according to the present disclosure may be through a known method of multivariate analysis. The method of multivariate analysis on the entire spectrum may be proceeded by, for example, commonly used Partial Least Squares-Discriminant Analysis (PLS-DA), General Discriminant Analysis (GDA), Principal Component Analysis (PCA), Parallel Factor Analysis (PARAFAC), Neaural Network Analysis (NNA) and/or Support Vector Machine (SVM) although not limited thereto.

Although not limited thereto, a diagnosis method of cancer according to one embodiment may comprise the following steps, removing background signal about the obtained spectrum; obtaining latent variable by performing PLS-DA on the entire spectrum, distinguishing two groups entered based on the variables, and verifying reliability thereof.

As above, the present disclosure allows accurate and quick on-site diagnosis of cancer. Especially, diagnosis accuracy for prostate cancer and pancreatic cancer was shown to be more than 90%.

According to another aspect, a preparation method of a substrate for spectroscopic analysis is a method of preparing a substrate for spectroscopic analysis described in the present disclosure and comprises a) preparing a base member; and b) forming a polycrystalline structure by soaking the base member in a composition for preparing a substrate for spectroscopic analysis. Detailed descriptions of explanation on the above substrate for spectroscopic analysis and redundant parts are omitted.

Step a) is preparation of a base member. As stated above, the present disclosure may involve various base members. Although not limited thereto, base member composed of at least one species of a paper, a polymer, a well plate, a wafer, and a protein may be prepared.

Step b) is formation of a polycrystalline structure by soaking the base member in a composition for preparing a substrate for spectroscopic analysis. The composition for preparing a substrate for spectroscopic analysis comprises a solution of a noble metal precursor and a reducing agent, thereby forming a polycrystalline structure having a plurality of grain boundaries composed of a cluster of a plurality of nanoparticles directly on the base member through a liquid process.

A ratio of a reducing agent to a noble metal precursor solution in the step b) may be set as 1:0.5 to 1:15 so that a step of shape controlling for controlling a shape of the polycrystalline structure may be comprised. By controlling an optimum ratio of a reducing agent according to a reducing agent type, a shape of a polycrystalline structure formed on a substrate for spectroscopic analysis may be easily controlled. In addition, by controlling the time for soaking the base member in a composition for preparing a substrate for spectroscopic analysis, a shape of a polycrystalline structure formed on a substrate for spectroscopic analysis may be easily controlled. Through a single process of soaking once, a preparation method of a substrate for spectroscopic analysis of the present disclosure may enhance signal intensity and signal uniformity at once.

Although not limited thereto, the step a) of a preparation method of a substrate for spectroscopic analysis of the present disclosure may further comprise surface modifying for surface modification of surface of the base member prior to forming a polycrystalline structure. The surface modifying, although not limited thereto, may involve controlling particle formation through chemical treatment of surface, or increasing particle density when particles are formed after surface modification.

Although not limited thereto, the surface modifying in the preparation method of a substrate for spectroscopic analysis of the present disclosure may comprise treating the base member surface with a surface modification composition comprising at least one species selected from 1 to 3 % base and 1 to 3% acid, and C₁₋₅ alcohol or water as solvent. Although not limited thereto, it may be suitable for enhancement of signal intensity and signal uniformity that 1% base or acid is used, and ethanol is used as solvent.

(a) in FIG. 19 and (b) in FIG. 19 illustrate surface modification results obtained by using a surface modifying composition according to one embodiment of the present disclosure. That is, (a) in FIG. 19 is a SEM photograph illustrating a result of increased particle density through surface modification using a surface modifying composition according to one embodiment of the present disclosure, and (b) in FIG. 19 is a graph illustrating a result of enhanced signal uniformity accordingly. Therefore, it can be verified that particle density has increased by using a surface modifying composition according to one embodiment, and that signal uniformity has been enhanced by the increased particle density.

### Embodiment

### 1. Preparation of Substrate for Spectroscopic Analysis

A base member in a form of a paper, a 3-dimension swab stick composed of a porous polymer, a wearable patch, and a film was prepared. A composition for preparing a substrate for spectroscopic analysis was prepared by mixing in a ratio of a reducing agent solution to a noble metal precursor solution as 5:1, 2:1, or 1:1 to 1:15. Only, a base member should be soaked within 1 minute from preparation of the composition. However, for a well plate, it is prepared by soaking a composition in each well of a well plate. Polycrystalline structures formed of a cluster of a plurality of nanoparticles on the base member are formed by soaking the prepared base member in the mixed composition for preparing a substrate for spectroscopic analysis for 30 seconds to 24 hours. HAuCl₄ was used as a noble metal precursor solution in the composition for preparing the substrate, and hydroxylamine, ascorbic acid, FeSO₄, and hydroxyquinone were used as a reducing agent. After formation of a polycrystalline structure, remaining precursor and a reducing agent were removed by washing with either water or ethanol twice. Then, after drying in a room temperature for at least one hour, the preparation of the substrate for spectroscopic analysis was completed.

### Result

Regardless of a composition and a form of a base member, such as forms of a paper, a 3-D swab stick composed of porous sponge, a patch, and a film, as a ratio of a reducing agent to a noble precursor solution included in a composition for preparing a substrate for spectroscopic analysis increased, a size of a prepared polycrystalline structure clustered on a substrate for spectroscopic analysis decreased and density of polycrystalline structures increased.

Accordingly, transmittance decreased, and light absorbance increased. In addition, polycrystalline structures showed majority of distribution with an average diameter in the range of 1 µm to 100 µm. Depending on a type of a reducing agent and based on SERS sensitivity and SERS signal intensity, an optimum range of a reducing agent for a noble metal precursor was obtained.

Table. 1 below shows optimum ratio ranges of a reducing agent to a noble metal precursor depending on a type of a reducing agent.

**Table 1**

| **Reducing Agent Type** | **Optimum Ratio Range of Reducing Agent** |
|---|---|
| Hydroxylamine (HA) | At least 1:1 less than 1:5 |
| Ascorbic acid (AA) | 1:0.5 to 1:15 |
| FeSO₄ | More than 1:1 and at most 1:15 |
| Hydroxyquinone (HQ) | 1:0.5 to less than 1:15 |

Specific parts of the present disclosure are described in detail above. It will be clear to a person ordinarily skilled in the art that the specific descriptions are merely preferable embodiments and that the scope of the present disclosure is not limited thereto. Accordingly, it should be noted that practical scope of the present disclosure is defined by the attached claims and equivalents thereof.

## Claims

1. A substrate for spectroscopic analysis comprising a base member; and
a polycrystalline structure composed of a cluster of a plurality of nanoparticles formed on the base member to have a plurality of grain boundaries, wherein the substrate for spectroscopic analysis is substrate formed by a solution process.

2. A substrate for spectroscopic analysis according to claim 1, wherein the base member is
composed of at least one species of a paper, a polymer, a well plate, a wafer, and a protein.

3. A substrate for spectroscopic analysis according to claim 1, wherein the nanoparticles are interconnected in a branch form, and wherein the polycrystalline structure has an average diameter of 1µm to 100µm.

4. A substrate for spectroscopic analysis according to claim 1, wherein an electrochemically active surface area (EASA) is 0.1 cm² to 10.0 cm².

5. A substrate for spectroscopic analysis according to claim 1, wherein the nanoparticles are composed of at least one species of Au, Ag, and Pt.

6. A substrate for spectroscopic analysis according to claim 1, wherein the substrate for spectroscopic analysis is applicable to a Raman spectroscopy system using a wavelength of at least 500 nm or a near-IR FT Raman spectroscopy system using a wavelength of 1064nm.

7. A substrate for spectroscopic analysis according to claim 1, wherein the substrate for spectroscopic analysis is composed of a 3-dimension swab stick, a wearable substrate, or a plasmonic well plate.

8. A composition for preparing a substrate for spectroscopic analysis comprising a noble metal precursor and a reducing agent, wherein a ratio of the reducing agent to the noble metal precursor is 1:0.5 to 1:15.

9. A composition for preparing a substrate for spectroscopic analysis according to claim 8, wherein, as the ratio of the reducing agent to the noble precursor increases, a size of a polycrystalline structure formed on a substrate for spectroscopic analysis decreases and density of a polycrystalline structure increases.

10. A composition for preparing a substrate for spectroscopic analysis according to claim 8, wherein the noble metal precursor is at least one species of HAuCl₄ and NaAuCl₄.

11. A composition for preparing a substrate for spectroscopic analysis according to claim 8, wherein the reducing agent is at least one species of hydroxylamine, ascorbic acid, FeSO₄, and hydroxyquinone.

12. A Raman spectroscopy device comprising a light source;
a substrate for spectroscopic analysis according to claim 1; and
a detector for detecting Raman scattering.

13. A Raman spectroscopy device according to claim 12 further comprising:
an adaptor provided with a first end in an open cylindrical form, the first end provided with a joint unit fastening to the Raman spectroscopy device, and a second end, the second end provided with a coupling unit formed with a through hole inducing light from a light source; and
a measurement unit in a spoon shape composed of a head unit and a straight body unit extended from the head unit, the head unit provided with a hemispherical seating unit to which the coupling unit of the second end of the adaptor is placed and a seating groove extended from the center of the body unit to the hemispherical seating unit in a longitudinal direction so that a straight strip substrate for spectroscopic analysis is placed.

14. A Raman spectroscopy device according to claim 13, wherein the seating groove is formed in a T-shape so that an end of the strip substrate for spectroscopic analysis is prevented from deviating from the hemispherical seating unit.

15. A Raman spectroscopy device according to claim 12, wherein a target material of the detector may be selected from at least one species of a cell, a metabolite, a protein, a nucleic acid, DNA, RNA, an enzyme, an organic molecule, a virus, an extracellular vesicle, a microvesicle, an exosome, and fat in urine, saliva, sweat or tear.

16. A Raman spectroscopy device according to claim 12, wherein the Raman spectroscopy device allows on-site diagnosis of cancer.

17. A preparation method of a substrate for spectroscopic analysis of claim 1 comprising:
a) preparing a base member for preparation of a base member; and
b) forming a polycrystalline structure with a plurality of grain boundaries composed of a cluster of a plurality of nanoparticles on the base member by soaking the base member in a composition for preparing a substrate for spectroscopic analysis comprising a solution of a noble metal precursor and a reducing agent,
wherein the step b) comprises controlling shape of the polycrystalline structure by having a ratio of a reducing agent to a noble metal precursor solution of 1:0.5 to 1:15.

18. A preparation method of a substrate for spectroscopic analysis according to claim 17,
wherein the step a) further comprises surface modifying for surface modification of the base member surface prior to formation of a polycrystalline structure.

19. A preparation method of a substrate for spectroscopic analysis according to claim 18,
wherein the surface modifying comprises treating the base member surface with a surface modification composition comprising at least one species selected from 1 to 3% base and 1 to 3% acid, and C₁₋₅ alcohol or water as solvent.
